# EUROPEAN PATENT SPECIFICATION

(11) **EP 3 939 691 B1**
(45) Date of publication and mention of the grant of the patent: **22.11.2023**
(21) Application number: 20185516.0
(22) Date of filing: 13.07.2020
(51) Int. Cl.: B01D 61/14, A61K 47/68, B01D 61/24, B01J 19/24

(54) **DEVICE ASSEMBLY FOR PRODUCING BIOCONJUGATES**
VORRICHTUNGSANORDNUNG ZUR HERSTELLUNG VON BIOKONJUGATEN
DISPOSITIF D'ASSEMBLAGE DE PRODUCTION DE BIO-CONJUGUÉS

(43) Date of publication of application: 19.01.2022
(73) Proprietor: Sartorius Stedim Biotech GmbH, 37079 Göttingen (DE)
(72) Inventor: DREHER, Thomas, 37079 Göttingen (DE); ESPACHS BARROSO, Alexandre, 37079 Göttingen (DE); GOHS, Michael, 37079 Göttingen (DE); HALT, Alexander, 34302 Guxhagen (DE); NICKEL, Björn, 34302 Guxhagen (DE)
(74) Representative: Novagraaf International SA

(56) References cited:
- WO-A1-96/40733
- WO-A1-2019/016067
- WO-A1-2019/016070
- WO-A2-03/057163
- US-A1- 2005 175 619
- US-A1- 2019 270 769

## Description

The invention relates to a device assembly for producing bioconjugates, in particular antibody-drug conjugates.

Synthesis of bioconjugates (bioconjugation) is a chemical strategy to establish stable covalent bonds between a biomolecule and another molecule or material. Especially, molecules that are undergoing some type of biospecific affinity interaction within cells can be linked together and "frozen" in the act of binding to each other with the use of crosslinking agents. This technology represents a new trend especially in the pharmaceutical industry since the biomolecules are able to recognize certain surface structures in organisms and thus can bring a coupled active ingredient to specific locations in the organism.

A particular class of biopharmaceutical drugs produced by bioconjugation are antibody-drug conjugates (ADCs) which are designed as a targeted therapy for treating cancer. ADCs are complex molecules composed of a monoclonal antibody linked to a biologically active cytotoxic payload or drug (toxin). They can be designed to distinguish between healthy and diseased tissue. Thus, unlike chemotherapy, ADCs are capable of killing tumor cells while sparing healthy cells.

In the production of ADCs, at present, glass or stainless steel vessels are used for the conjugation reaction, i.e. for coupling the toxin to the biomolecule. These vessels are capable of ensuring the desired reaction conditions, such as a certain constant temperature. However, the use of reusable components in a process device is disadvantageous in that between two uses of the process device an intensive cleaning and further preparations are required which are time-consuming and involve high costs. Moreover, during cleaning it is possible that the operator of the process device comes into contact with remaining toxins, thus exposing him or her to a high health risk.

For changing the buffer or concentrating the bioconjugate, the glass or stainless steel vessels are usually combined with other process devices. This constitutes an obstacle to automation and process safety since a plurality of different devices and process steps are involved.

US 2005/0175619 A1 discloses a method of producing an antibody conjugate with at least one effector moiety based on a conjugation reaction in a solution comprising at least 5% by volume of an alcohol. The antibody conjugation process, including antibody reduction step and final filtration and formulation steps of conjugate product, are performed in a "single-pot" enclosed recirculation/ ultrafiltration apparatus. However, this would not be possible if, instead of alcohol, another common solvent was used, because lumps would form during the conjugation which would block the filter membrane in the filtration process following the conjugation reaction.

US 2019/0270769 A1 shows how unit operations can be combined to create a continuous ADC production and processing method. In particular, single-pass tangential flow filtration or countercurrent diafiltration is linked to conjugation reactions to facilitate the concentration of ADCs and also to remove unconjugated products from the conjugation reaction and exchange the purified ADC into a formulation buffer. Such a continuous process is not suitable for conjugation reactions in solvents where lumps would form and block the filter membrane during concentration of the ADC.

A device assembly for producing bioconjugates according to the preamble portion of claim 1 is known from WO 2019/016067 A1. A conjugation unit comprises a reservoir containing buffer solution, a reservoir containing a toxophore, a mixing device, a residence time device, two homogenization loops and several pumps for controlling the dosage of the different fluid streams, two waste outlets and a reservoir containing buffer solution for the subsequent unit operation. A subsequent unit for continuous ultrafiltration comprises an ultrafiltration device, a depth filter, a waste outlet, another filter, a detection device, a surge bag and several pumps for controlling the fluid flow. The process can be controlled by a PCS7 process system.

It is an object of the invention to overcome the above drawbacks and to enable a more efficient and safe production of bioconjugates, especially ADCs, using common solvents.

The above problem is solved by a device assembly for producing bioconjugates according to claim 1. Advantageous and expedient embodiments of the invention are apparent from the dependent claims.

The invention provides a device assembly for producing bioconjugates, in particular antibody-drug conjugates, comprising a conjugation unit for performing a bioconjugation reaction in a medium, a first filtration unit for separating precipitates and/or agglomerates, and a second filtration unit for performing an ultrafiltration (UF) and/or a diafiltration (DF) process. The first filtration unit is arranged in a flow path between the conjugation unit and the second filtration unit. The device assembly further comprises a single control unit for controlling the transfer of medium from the conjugation unit through the first filtration unit to the second filtration unit and for controlling the ultrafiltration and/or diafiltration process.

The invention combines three major unit operations of a bioconjugate production process in a single device assembly. Thanks to the control unit it is possible to enable a highly automated batch process, including a bioconjugation reaction step yielding bioconjugate, a filtration step for separating undesired precipitates and/or agglomerates from the medium containing the bioconjugate, and an UF/DF process for purifying and concentrating the bioconjugate. The filtration step after the bioconjugation reaction and before the UF/DF process ensures that the filter membranes of the second filtration unit will not become clogged by the precipitates and/or agglomerates. Thanks to the automation, no human interaction is necessary at the device assembly during the process. Thus, the device assembly (except for an operating panel) can be placed behind a glass pane, under a fume hood or in an isolator (isolated room) separated from the operator. This significantly increases the safety of the operating personnel in view of the toxic substances involved.

In accordance with the current trend towards single-use concepts in the pharmaceutical process development and production, it is preferred that those components of the device assembly which come into contact with medium during the process, hereinafter referred to as wetted components, are configured as single-use components. The single-use components are disposed of after use so that there is no costly and time-consuming effort of cleaning, checking and validating the components for their next use. Considering the highly toxic substances involved in ADC production, this is highly appreciated.

However, a major challenge with respect to the use of single-use components is the fact that the solvents that are typically used in bioconjugate production cause (at least partial) dissolution of many materials that single-use components are typically made of. Such solvents include dimethylacetamide (DMAc), dimethyl sulfoxide (DMSO), dimethylformamide (DMF), propylene glycol (PG), acetonitrile (ACN) and n-methyl-2-pyrrolidone (NMP).

It is to be noted that initially the solvent concentration is very high, while the supply of other fluids/solutions during the conjugation reaction and the further process steps reduces the solvent concentration. Therefore, a first group of wetted components of the device assembly that come into contact with medium having a very high solvent concentration are preferably configured as single-use components made from one or more materials of a first group of materials consisting of: high-density polyethylene (HDPE); polyamide (PA); polybutylene terephthalate (PBT); polytetrafluoroethylene (PTFE); polypropylene (PP); polyethylene terephthalate (PET); ceramics; glass. These materials are resistant to a 100% concentration of the above-mentioned solvents. For example, a line connecting a toxin reservoir with a reaction vessel of the conjugation unit has to be made of such a material.

A second group of wetted components of the device assembly that come into contact with medium having a lower solvent concentration are preferably configured as single-use components made from one or more materials of a second group of materials consisting of: low-density polyethylene (LDPE); (reinforced) platinum cured silicone (Si(Pt)); thermoplastic elastomer (TPE); thermoplastic polyolefin (TPO); thermoplastic vulcanizate (TPV); ethylene propylene diene monomer rubber (EPDM); silicone. These materials are resistant at least to a 20 % concentration of the above-mentioned solvents. Of course, the second group components can also be made from a material of the first group of materials which are 100 % resistant.

Irrespective of the above, not all of the wetted components of the device assembly need to be made from the above-mentioned materials. Especially the filter membranes used in the filtration units of the device assembly can be made from one or more of the following materials, which are all resistant to a 100% concentration of the solvents mentioned further above: nylon; polytetrafluoroethylene (PTFE); polypropylene (PP) fleece. Commercially available Hydrosart^{®} high performance membranes (stabilized cellulose based membranes) are at least resistant to a 20 % concentration of those solvents.

The single-use components of the device assembly are preferably pre-sterilized so that bioburden testing before use of the components is not necessary.

According to a preferred embodiment of the device assembly, the conjugation unit comprises a flexible conjugation bag where the conjugation reaction takes place. The conjugation bag is held by a first bag holder.

In view of the high commercial value of the product, it is generally desired that after each process step as little medium as possible remains in the respective components of the device assembly. Therefore, when the conjugation reaction is terminated, the conjugation bag should be emptied as far as possible. This is facilitated by a bag holder having a bottom surface and a draining opening formed at a lowest point of the bottom surface. Accordingly, the conjugation bag has a bottom portion resting on the bottom surface of the first bag holder such that an outlet formed in the bottom portion of the conjugation bag is located at the draining opening of the first bag holder. With this configuration a draining line can be easily connected to the low-positioned outlet of the conjugation bag. It is particularly preferred that the draining opening in the bottom surface of the first bag holder has a conical shape. The main advantage of such a design is a minimum hold-up volume. During the draining process the medium level is kept higher due to the conical draining opening. Accordingly, a stirrer stays in contact with the medium longer. This results in a better stirring performance especially at low volumes.

A temperature control system for controlling the temperature of the medium in the conjugation bag can be used to provide optimum reaction conditions. According to a preferred construction, the first bag holder includes a double-walled containment, through which a heat transfer fluid flows. It is thus possible to adjust the temperature in the conjugation bag as desired.

A pH sensor arranged in the conjugation bag and coupled to the control unit of the device assembly enables pH monitoring during the conjugation reaction. The pH measurements provide information about the progress of the conjugation reaction and can be used to determine when a sufficient amount of bioconjugate has been formed and the conjugation reaction can be terminated. Further stop criterions can be involved in this decision.

When the conjugation reaction is terminated, the medium of the conjugation bag needs to be transferred to the second filtration unit for purification and concentration of the product. For an automated transfer, the conjugation unit preferably comprises a controllable draining valve allowing the medium to drain from the conjugation bag. Further, the first filtration unit comprises a transfer pump for transferring the medium from the conjugation unit through the first filtration unit to the second filtration unit. Both the controllable valve and the transfer pump are coupled to the control unit of the device assembly.

Before the UF/DF filtration, the filter device of the first filtration unit removes the precipitates and/or agglomerates from the medium so that they cannot block the filter membranes used for purification and concentration of the product in the second filtration unit. The membrane of the filter device of the first filtration unit has a pore size of less than 0.8 µm, preferably about 0.2 µm or less. Such membranes are commonly used for sterile filtration. Of course, the pore size must not be smaller than the diameter of the bioconjugate.

According to the invention, the second filtration unit comprises a single-use loop-assembly for recirculating the medium in the ultrafiltration and/or diafiltration process. The single-use loop-assembly can be mounted to and/or dismounted from a basic structure of the device assembly as a whole, preferably together with a single-use conjugation bag and a single-use recirculation bag and other single-use components of the device assembly. The single-use loop-assembly is preconfigured and, if possible, can already be preassembled before it is mounted to a basic structure of the device assembly. This significantly facilitates the handling of the components used in the UF/DF process, and false connections are ruled out from the outset. Furthermore, the design of the single-use loop-assembly can be optimized with respect to the dead volume (hold-up volume). A small dead volume allows a more effective purification and concentration and less product loss.

The single-use loop-assembly preferably includes a recirculation pump, at least one sensor, tubing and connectors, which - together with a recirculation bag - are major components of a regular UF/DF filtration assembly. It is generally preferred that all single-use components that had contact with medium can be dismounted as one single unit (the single-use loop assembly plus further wetted single-use components).

As mentioned before, as little medium as possible should remain in the respective components of the device assembly. Similar to the conjugation bag and the first bag holder, the recirculation bag is preferably held in a second bag holder having a bottom surface and a draining opening formed at a lowest point of the bottom surface. Accordingly, the recirculation bag has a bottom portion resting on the bottom surface of the second bag holder such that an outlet formed in the bottom portion of the recirculation bag is located at the draining opening of the second bag holder. With this configuration a draining line can be easily connected to the low-positioned outlet of the recirculation bag.

A temperature control system can be used to control the temperature of the medium in the recirculation bag. According to a preferred construction, the second bag holder includes a double-walled containment, through which a heat transfer fluid flows. It is thus possible to adjust the temperature in the recirculation bag as desired.

A conductivity sensor arranged in the recirculation bag and coupled to the control unit of the device assembly enables monitoring of the conductivity of the circulating medium during the UF/DF filtration process. The conductivity measurements provide information about the purity and concentration of the bioconjugate in the circulated medium. A predetermined conductivity value can be set as a threshold for determining when the UF/DF filtration process is to be terminated. Further stop criterions can be involved in this decision, e.g. based on pH or volume of the permeate.

Due to the high toxicity of the substances involved in bioconjugate production, especially in ADC production, a very high safety standard has to be guaranteed. In particular, any contact of toxic medium with the operator of the device assembly or with the environment is to be avoided. In view of these requirements, especially the single-use loop-assembly includes controllable valves, preferably pinch valves, allowing an automated change of flow paths without human interaction. It is thus possible to let the control unit of the device assembly control the flow of the medium as required in the respective process steps. This allows the device assembly (except for an operating panel) to be placed in a restricted area remote from the operator.

The controllable valves of the device assembly are even more advantageous. Before dismounting and disposal of the single-use loop-assembly together with the other single-use components (conjugation bag, recirculation bag, tubing, connectors etc.) as a whole, the valves can be controlled such that they create a confinement for the residual medium. Since it is safely captured, neither the operator nor the environment can come into contact with the toxic medium during dismounting and removal.

In a preferred embodiment of the device assembly, a dip tray is arranged below the single-use loop-assembly to further increase the safety standard. The dip tray has a bottom surface and a draining means formed at a lowest point of the bottom surface so that any medium leaking from the single-use loop-assembly can be collected in a waste tank placed below the draining means. The draining means can be a simple hole or a valve, e.g. a manual ball valve.

The second filtration unit, where the UF/DF process is performed, preferably comprises at least one cross-flow filter cassette held in a filter press. The filter press allows great flexibility with respect to the type, number and arrangement of filter cassettes.

The control unit of the device assembly is preferably configured to control at least one of the following: temperature of the heat transfer fluid flowing through the double-walled first bag holder; temperature of the heat transfer fluid flowing through the double-walled second bag holder; the controllable valves of the conjugation unit and/or the second filtration unit, especially the valves of the single-use loop-assembly; the transfer pump; the recirculation pump.

For easier handling of the whole device assembly, especially with respect to moving it into a restricted area for operation, all components of the device assembly, except for an operating panel coupled to the control unit, can be mounted on a trolley. The mechanically decoupled operating panel allows the operator to make any adjustments in a safe place remote from the device assembly.

Further features and advantages of the invention will become apparent from the following description and from the accompanying drawing to which reference is made. In the drawings:
- Figure 1 shows a device assembly for producing bioconjugates according to the invention without the most parts of a single-use loop-assembly; and
- Figure 2 shows another view of the device assembly of Figure 1 with the single-use loop-assembly;
- Figure 3 shows a single-use conjugation bag and equipment used in the device assembly;
- Figure 3a shows a detail of the conjugation bag;
- Figure 4 shows a contactless single-use stirrer of the impeller system of the device assembly; and
- Figure 5 shows a single-use recirculation bag and equipment used in the device assembly.

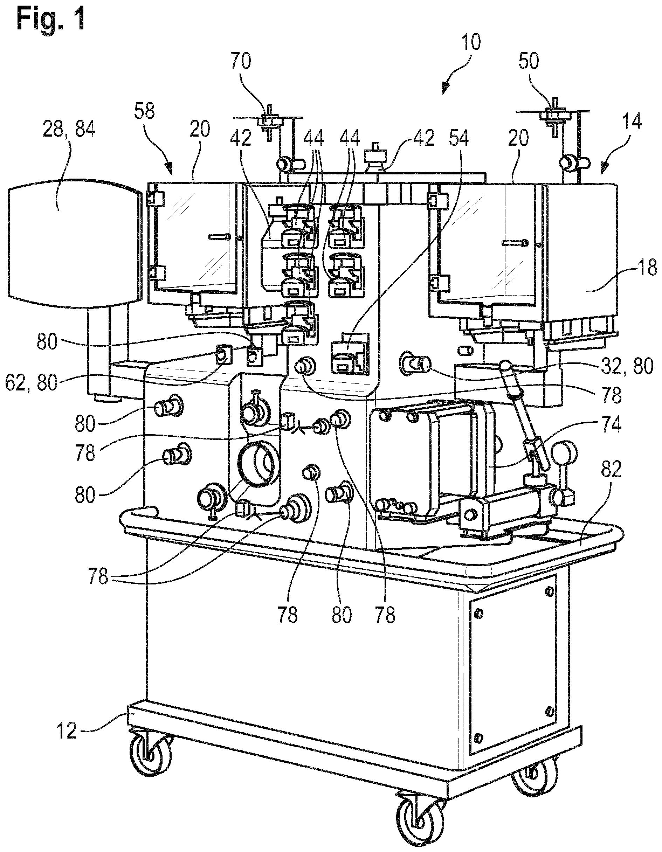

As shown in Figure 1, an entire device assembly 10 for producing bioconjugates is built on a trolley 12 and movable as a whole. In the device assembly 10 three dedicated units for performing three unit operations in a bioconjugate production process are combined. The first unit is a conjugation unit for performing a bioconjugation reaction in a medium. The second unit is a filtration unit for separating precipitates and/or agglomerates from the medium containing the bioconjugate. The third unit is a filtration unit for performing an ultrafiltration and/or a diafiltration process for purifying and concentrating the bioconjugate product. These three units will be described in detail below.

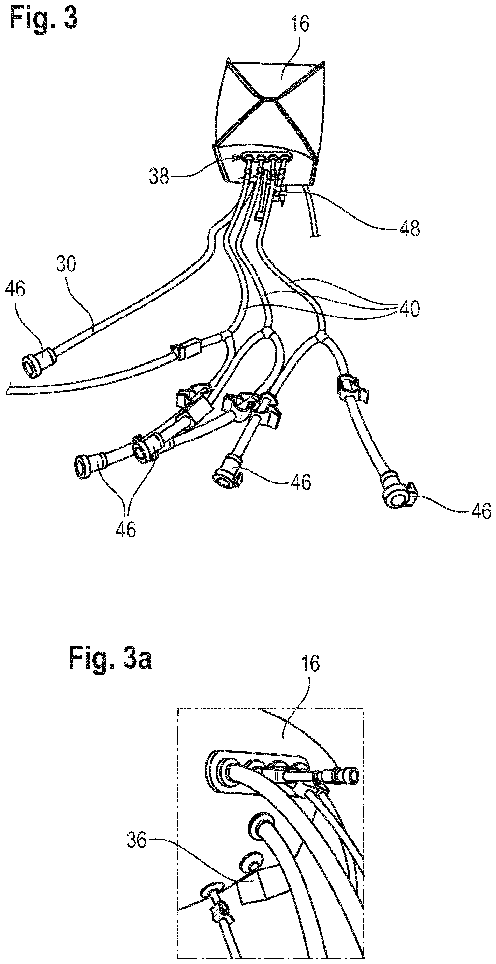

The conjugation unit comprises a first bag holder 14 for accommodating a flexible single-use conjugation bag 16 (shown separately in Figure 3). The first bag holder 14 includes a double-walled or double-jacketed containment 18 and a transparent glass door 20. The containment 18 together with the glass door 20 laterally surround a receiving space. The glass door 20 enables easy access and makes the receiving space visible to the operator. While its upper side is open, the first bag holder 14 has a conical or otherwise inclined bottom surface 22 with a draining opening 24 formed at the lowest point of the bottom surface 22. The first bag holder 14 further includes a load cell 26 for measuring the weight of the medium in the conjugation bag 16. The load cell 26 is connected to a central control unit 28 of the device assembly 10 that will be described in more detail later.

The conjugation bag 16 (see Figure 3) is a 3D-shaped sterilized single-use bag made from a flexible foil material. The volume of the conjugation bag 16 and the dimensions of the first bag holder 14 are adapted to each other so that the filled conjugation bag 16 takes a stable position in the receiving space. A typical working volume of the conjugation bag 16 is 20 L. Of course, other volumes are possible.

Regardless of the size, a bottom portion of the conjugation bag 16 rests on the bottom surface 22 of the first bag holder 14 such that a lower outlet port of the conjugation bag 16 is located directly above or in the draining opening 24. The lower outlet port is connected to a conjugation outlet hose line (draining line) 30 through the draining opening 24. The conjugation outlet hose line 30 can be opened and blocked by a draining valve 32, preferably a pinch valve, which is controlled by the control unit 28. Since the lower outlet port is placed at the lowest point of the bottom surface 22, the conjugation bag 16 can be completely emptied by gravity without any active draining means when the conjugation outlet hose line 30 is opened by the draining valve 32.

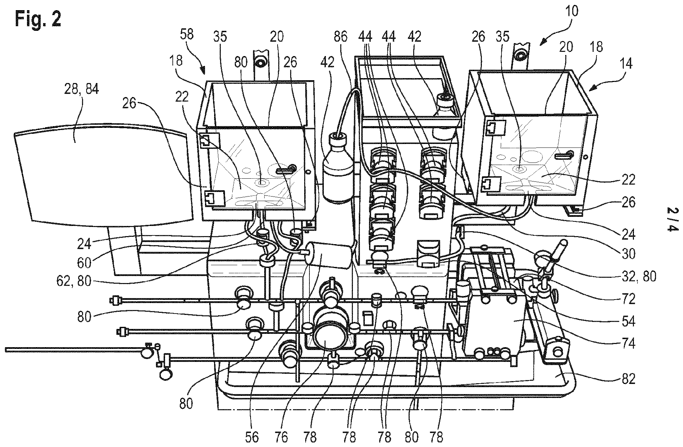

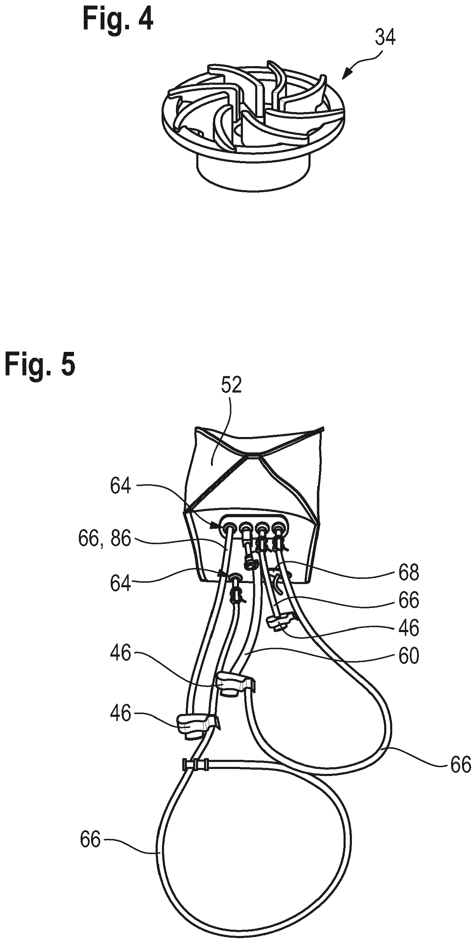

The conjugation unit comprises an impeller system (not visible in Figures 1 and 2). At the bottom of the first bag holder 14 a magnetic drive causes a contactless stirrer 34 (shown separately in Figure 4), which is placed loosely inside the conjugation bag 16, to lift from a receptacle 35 and rotate. In Figure 3a the magnetic coupling 36 between the magnetic drive and the stirrer 34 can be seen. The stirring of the medium ensures a homogeneous mixture throughout the conjugation reaction. The contactless stirrer 34 produces only little abrasion inside the conjugation bag 16. However, other impeller systems can be used as well, e.g. an impeller system including a driven shaft penetrating into the conjugation bag 16 through a sealed opening. The impeller system is controlled by the control unit 28.

As can be seen in Figures 3 and 3a, the conjugation bag 16 is provided with several ports 38 for connecting supply lines 40 through which the fluids/solutions necessary for the conjugation reaction are fed into the conjugation bag 16. Peristaltic pumps 44 (see Figures 1 and 2) controlled by the control unit 28 are used to supply the fluids/solutions as required from reservoirs 42 or other sources through the supply lines 40, which are connected to the ports 38 of the conjugation bag 16 on the one hand, and to the reservoirs 42 or the other sources on the other hand, via sterile connectors 46.

The conjugation bag 16 is equipped with a single-use pH sensor 48 (probe). Other single-use sensors for detecting parameters of interest in connection with the conjugation reaction can be provided as well. The sensors are connected to the control unit 28.

The conjugation unit also comprises a first temperature control system coupled to the control unit 28 for controlling the temperature of the medium in the conjugation bag 16. A heat transfer fluid can be pumped through the double-walled containment 18 of the first bag holder 14 to cool or heat the medium in the conjugation bag 16 as desired. A temperature sensor transmits the current temperature value to the control unit 28. The temperature sensor can be arranged at the bottom surface 22 of the first bag holder 14, for example.

Further, a first sterile venting filter 50 (see Figure 1) for venting the conjugation bag 16 is fixed to a mount above the conjugation bag 16 and connected to a venting port of the conjugation bag 16.

The conjugation outlet hose line 30 is part of a flow path extending between the conjugation bag 16 and a recirculation bag 52 of a second filtration unit for performing an ultrafiltration and/or a diafiltration process which will be described later. A peristaltic transfer pump 54 and a single-use filter device 56 are integrated into this flow path, forming a first filtration unit of the device assembly 10.

The general type and the specific configuration of the filter device 56 can be chosen according to the given requirements of the separation process. In the embodiment shown in Figure 2 the filter device 56 is a filter capsule having a multilayer membrane made of one or more of the following materials: nylon; polytetrafluoroethylene (PTFE); polypropylene (PP) fleece. The membrane may also be a stabilized cellulose based membrane, e.g. a Hydrosart^{®} high performance membrane.

The membrane of the filter device 56 has a pore size of less than 0.8 µm. Preferably, the filter device 56 is a sterile filter with a pore size of about 0.2 µm or less, which is yet larger than the size of the bioconjugate formed in the medium of the conjugation bag 16. Thus, the filter device 56 is capable of effectively separating precipitates and/or agglomerates (or other unwanted lumps) from the medium containing the bioconjugate that have also formed unintentionally during the conjugation reaction in the conjugation bag 16.

The second filtration unit of the device assembly 10 comprises the already mentioned recirculation bag 52 (shown separately in Figure 5) which is held in a second bag holder 58. The recirculation bag 52 and the conjugation bag 16 are of the same or a similar type and size.

In a bottom portion of the recirculation bag 52 a lower outlet port is connected to a recirculation outlet hose line (draining line) 60 which can be opened or closed by a draining valve 62, preferably a pinch valve (see Figure 2), controlled by the control unit 28. Further, the recirculation bag 52 has several ports 64, one of which is for receiving the filtrate of the medium that passes through the filter device 56 of the first filtration unit. Other ports 64 of the recirculation bag 52 enable the supply or exchange of buffers or other fluids/solutions during the UF/DF filtration process. Similar to the conjugation bag 16, hose lines 66 are connected to the ports 64 of the recirculation bag 52 on the one hand, and to reservoirs or other sources or to further tubing of the device assembly 10 on the other hand, via sterile connectors 46.

Instead of or in addition to a single-use pH sensor as used in the conjugation bag 16, the recirculation bag 52 is equipped with a single-use conductivity sensor 68. Other single-use sensors for detecting parameters of interest in connection with the filtration process running in the second filtration unit can be provided as well. The sensors are connected to the control unit 28.

Like the conjugation bag 16 and the recirculation bag 52, also the first bag holder 14 and the second bag holder 58 have a similar design, including - inter alia - a draining opening 24 at the bottom surface 22 for the recirculation outlet hose line 60 and a load cell 26 connected to the control unit 28. A second sterile venting filter 70 (see Figure 1) for venting the recirculation bag 52 is fixed to a mount above the recirculation bag 52 and connected to a venting port of the recirculation bag 52. The temperature of the medium in the recirculation bag 52 is controlled by a second temperature control system similar to the first temperature control system. However, an impeller system is not required for the medium in the recirculation bag 52.

The recirculation outlet hose line 60 is part of a tubing of the second filtration unit leading to one or more single-use filter cassettes 72 held in a filter press 74. The filter cassettes 72 are self-contained units configured for cross-flow filtration, in particular for an ultrafiltration or diafiltration process. Several filter cassettes 72 can be connected in parallel or in series.

The type, number and arrangement of filter cassettes 72 can be chosen according to the given requirements of the UF/DF process. The nominal molecular weight limit (NMWL) or the molecular weight cutoff (MWCO) of the membranes of the filter cassettes 72 is adapted to the size and weight of the bioconjugate so that the unbound small-sized and lighter components of the medium are washed out to the permeate side while the larger-sized and heavier bioconjugate is kept in the retentate. The total membrane area of the filter cassette(s) 72 preferably ranges from 0.1 to 1.4 m².

The second filtration unit further comprises a single-use recirculation pump 76, tubing, connectors, sensors 78 and further controllable valves 80, preferably pinch valves, enabling the blocking and unblocking of a variety of flow paths. Such flow paths include the path for recirculating the medium in the second filtration unit, and can further include paths for supplying buffer to the recirculation bag 52, discharging waste medium, flushing etc. The sensors 78 are pressure sensors and/or flow meters arranged in the feed, permeate and retentate lines of the tubing. An optional UV probe can be arranged in the permeate line for UV spectroscopy in order to detect any product in the permeate line in case of a defective membrane. Like the conductivity sensor 68 in the recirculation bag 52, the sensors 78 transmit their measured values or signals to the control unit 28. The pinch valves 80 are all controlled by the control unit 28.

Thus, the second filtration unit is capable of performing a complete UF/DF process for purifying and concentrating the bioconjugate, controlled by the control unit 28 without human interaction.

A special characteristic of the second filtration unit is that the recirculation pump 76, the tubing, the connectors 46, the sensors 78 and the valves 80 used in the UF/DF process are single-use components configured as a preassembled unit, hereinafter referred to as single-use loop-assembly. The complete single-use loop-assembly can be mounted to and dismounted from a basic structure of the device assembly 10 as a whole.

The pre-configured tubing design of the single-use loop assembly ensures a minimum dead volume (hold-up volume), e.g. below 300 mL. This is very important in view of the high monetary value of the bioconjugate product.

Below the single-use loop-assembly a dip tray 82 is arranged. The dip tray 82 has a bottom surface and a draining means, e.g. a ball valve, formed at a lowest point of the bottom surface. Thus, in case of a leakage, the leaking fluid is collected in the dip tray 82 and can be guided to a waste tank positioned below the draining means.

The device assembly 10 further comprises an operating panel 84, preferably a touchscreen, which is part of the control unit 28. With the operating panel 84 an operator can make all selections and adjustments that are necessary before and after the bioconjugate production process, which itself is performed highly automatically. The operating panel 84 also serves to inform the operator of the current settings and status of the process and allows interaction, if necessary.

In the embodiment shown in Figures 1 and 2 the operating panel 84 is fixed to a mount of the device assembly 10. According to another embodiment, the operating panel 84 is a separate unit, wirelessly connected to the control unit 28 of the device assembly 10 or by a long enough cable, allowing a positioning of the operating panel 84 remote from the rest of the device assembly 10. The remote positioning of the operating panel 84 makes it possible to arrange the rest of the device assembly 10 in a restricted area, especially in an isolated room (isolator) and/or under a fume hood. If the possibility of human manipulation at the device assembly 10 is still required or desired, the device assembly 10 (without the operating panel 84) can be arranged in a glovebox.

All of the components that come into contact with toxic medium (wetted components) are dedicated single-use components that will not be used again and therefore need not be cleaned and sterilized after use. The wetted components are made from materials that are especially resistant to the following solvents which could be used in connection with the conjugation reaction: dimethylacetamide (DMAc), dimethyl sulfoxide (DMSO), dimethylformamide (DMF), propylene glycol (PG), acetonitrile (ACN), n-methyl-2-pyrrolidone (NMP).

A first group of the wetted components, especially wetted components of the conjugation unit, are resistant to a 100% concentration of the solvents (i.e. the pure solvents). This first group includes a toxin supply line 86 which leads from the reservoir 42 (here a bottle), containing toxin solved in 100% solvent, to the conjugation bag 16.

Since the mixing with other fluids/solutions during the conjugation reaction and the UF/DF filtration process dilutes the solvent, a second group of the wetted components, especially the wetted components of the second filtration unit, only have to be resistant to at least a 20 % concentration of the solvents. This also applies to the other supply lines 40 because they are connected to the conjugation bag 16 below the liquid level so that instant dilution occurs there.

The wetted components of the first and second groups may be made from one or more of the following materials (100 % resistant): high-density polyethylene (HDPE); polyamide (PA); polybutylene terephthalate (PBT); polytetrafluoroethylene (PTFE); polypropylene (PP); polyethylene terephthalate (PET); ceramics; glass. The wetted components of the second group may also be made from one or more of the following materials (20 % resistant): low-density polyethylene (LDPE); (reinforced) platinum cured silicone (Si(Pt)); thermoplastic elastomer (TPE); thermoplastic polyolefin (TPO); thermoplastic vulcanizate (TPV); ethylene propylene diene monomer rubber (EPDM); silicone.

In the following, operation of the device assembly 10 in a bioconjugate production process is described.

Before the device assembly 10 can be used, the single-use components are provided and mounted to the basic structure of the device assembly 10. In particular, the conjugation bag 16 and the recirculation bag 52 are placed in the respective bag holders 14, 58 and integrity tested, or the other way round. The single-use loop-assembly, including the recirculation pump 76, the tubing, the connectors 46, the sensors 78 and the valves 80 used in the UF/DF process, is mounted, step-by-step or as a whole, to the basic structure of the device assembly 10. The necessary connections between the single-use loop-assembly and the conjugation bag 16 and the recirculation bag 52 and any further connections are made via sterile connectors 46.

After the preparations, the operator starts the bioconjugate production process at the operating panel 84. The conjugation bag 16 is filled with the fluids/solutions necessary to start the conjugation reaction. The reaction conditions are monitored, and the peristaltic pumps 44 controlled by the control unit 28 supply fluids/solutions as required while the conjugation reaction takes place. The temperature in the conjugation bag 16 is adjusted via the first temperature control unit.

The conjugation reaction is terminated when the pH measured by the pH sensor 48 exceeds a predetermined threshold value. Further stop criterions may be involved, like expiry of a set time period or analysis of a sample taken from the conjugation bag 16. The control unit 28 then opens the draining valve 32 and activates the transfer pump 54.

The medium leaves the conjugation bag 16 through the conjugation outlet hose line 30 and passes through the filter device 56. Thereby the precipitates and/or agglomerates are separated from the filtrate. The filtrate containing the bioconjugate is directed to the recirculation bag 52.

When all of the filtrate has entered the recirculation bag 52, the UF/DF process for purifying and concentrating the bioconjugate in the second filtration unit is started. The control unit 28 activates the recirculation pump 76 to keep the medium circulating in the single-use loop-assembly with the filter cassettes 72. Here, the permeate with the unbound reactants is guided to a waste tank while the retentate with the bioconjugate is kept in the loop. Since the precipitates and/or agglomerates have been removed by the filter device 56, they cannot clog the membranes of the filter cassettes 72. Buffer is supplied/exchanged as required with the aid of the corresponding valves 80 which are controlled by the control unit 28.

The UF/DF process is terminated when the conductivity value measured by the conductivity sensor 68 in the recirculation bag 52 exceeds a predetermined threshold value. Further stop criterions may be involved, like expiry of a set time period or evaluation of a UV spectrum measured by the UV probe or analysis of a sample taken from the conjugation bag 16.

The control unit 28 controls the pinch valves 80 such that the retentate with the purified and concentrated bioconjugate can be extracted from the single-use loop-assembly through a harvest line into a container for further processing. Since the dead volume of the single-use loop-assembly is very low, only a small amount of the valuable medium remains in the loop-assembly.

Before the single-use loop-assembly, including the connected filter cassettes 72, is removed from the basic structure of the device assembly 10 together with the bags 16, 52, the tubing (including the supply and other hose lines 30, 40, 60, 66, 86), the connectors 46, the sensors 48, 68, 78 and the pinch valves 80 as a whole, the control unit 28 closes all peripheral pinch valves 80 to create a confinement for the residual medium in the loop-assembly. The sealed single-use loop-assembly together with the other single-use components is dismounted and disposed of as a whole, i.e. its components are not separated from each other. This enables a very high safety standard since the toxic medium cannot exit the loop-assembly and come into contact with the operator and the environment.

The device assembly 10 is particularly useful for preclinical or clinical production of ADCs.

The device assembly 10 can also be used without the conjugation unit and the first filtration unit, serving as a conventional UF/DF filtration device assembly with a disposable single-use loop-assembly.

### List of Reference Signs

- 10: device assembly
- 12: trolley
- 14: first bag holder
- 16: conjugation bag
- 18: containment
- 20: glass door
- 22: bottom surface
- 24: draining opening
- 26: load cell
- 28: control unit
- 30: conjugation outlet hose line
- 32: draining valve
- 34: stirrer
- 35: receptacle for stirrer
- 36: magnetic coupling
- 38: ports of conjugation bag
- 40: supply lines
- 42: reservoirs
- 44: peristaltic pumps
- 46: connectors
- 48: pH sensor
- 50: first venting filter
- 52: recirculation bag
- 54: transfer pump
- 56: filter device
- 58: second bag holder
- 60: recirculation outlet hose line
- 62: draining valve
- 64: ports of recirculation bag
- 66: hose lines
- 68: conductivity sensor
- 70: second venting filter
- 72: filter cassette
- 74: filter press
- 76: recirculation pump
- 78: sensors
- 80: controllable valves
- 82: dip tray
- 84: operating panel
- 86: toxin supply line

## Claims

1. A device assembly (10) for producing bioconjugates, in particular antibody-drug conjugates, the device assembly (10) comprising
a conjugation unit for performing a bioconjugation reaction in a medium,
a first filtration unit for separating precipitates and/or agglomerates,
a second filtration unit for performing an ultrafiltration and/or a diafiltration process,
the first filtration unit being arranged in a flow path between the conjugation unit and the second filtration unit;
the device assembly (10) further comprising a single control unit (28) for controlling the transfer of medium from the conjugation unit through the first filtration unit to the second filtration unit and for controlling the ultrafiltration and/or diafiltration process,
**characterized in that** the second filtration unit comprises a preconfigured single-use loop-assembly for recirculating the medium in the ultrafiltration and/or diafiltration process which can be mounted to and/or dismounted from a basic structure of the device assembly (10) as a whole.

2. The device assembly (10) according to claim 1, **characterized in that** a first group of components of the device assembly (10) coming into contact with the medium are configured as single-use components made from one or more materials of a first group of materials consisting of: high-density polyethylene (HDPE); polyamide (PA); polybutylene terephthalate (PBT); polytetrafluoroethylene (PTFE); polypropylene (PP); polyethylene terephthalate (PET); ceramics; glass; and **in that** a second group of components of the device assembly (10) coming into contact with the medium are configured as single-use components made from one or more of the following materials: low-density polyethylene (LDPE); (reinforced) platinum cured silicone (Si(Pt)); thermoplastic elastomer (TPE); thermoplastic polyolefin (TPO); thermoplastic vulcanizate (TPV); ethylene propylene diene monomer rubber (EPDM); silicone; a material from the first group of materials.

3. The device assembly (10) according to claim 1 or 2, **characterized in that** the conjugation unit comprises a flexible conjugation bag (16) and a first bag holder (14) holding the conjugation bag (16).

4. The device assembly (10) according to claim 3, **characterized in that** the conjugation bag (16) has a bottom portion and an outlet formed therein, the first bag holder (14) having a bottom surface (22) and a draining opening (24) formed at a lowest point of the bottom surface (22), the bottom portion of the conjugation bag (16) resting on the bottom surface (22) of the first bag holder (14) such that the outlet of the conjugation bag (16) is located at the draining opening (24) of the first bag holder (14).

5. The device assembly (10) according to claim 3 or 4, **characterized in that** the first bag holder (14) includes a double-walled containment (18), the conjugation unit further comprising a temperature control system for controlling the temperature of the medium in the conjugation bag (16), the temperature control system including a heat transfer fluid flowing through the double-walled containment (18) of the first bag holder (14).

6. The device assembly (10) according to any of claims 4 to 5, **characterized in that** a pH sensor (48) is arranged in the conjugation bag (16).

7. The device assembly (10) according to any of the preceding claims, **characterized in that** the conjugation unit comprises a controllable draining valve (32) and the first filtration unit comprises a transfer pump (54) for transferring medium from the conjugation unit through the first filtration unit to the second filtration unit.

8. The device assembly (10) according to any of the preceding claims, **characterized in that** the first filtration unit comprises a filter device (56) with a membrane having a pore size of less than 0.8 µm, preferably about 0.2 µm or less.

9. The device assembly (10) according to any of the preceding claims, **characterized in that** the single-use loop-assembly can be mounted to and/or dismounted from a basic structure of the device assembly (10) as a whole together with a single-use conjugation bag (16) and a single-use recirculation bag (52) and preferably other single-use components of the device assembly (10).

10. The device assembly (10) according to any of the preceding claims, **characterized in that** the single-use loop-assembly includes a recirculation pump (76), at least one sensor (78), tubing and connectors (46).

11. The device assembly (10) according to claim 10, **characterized in that** the recirculation bag (52) has a bottom portion and an outlet formed therein, the recirculation bag (52) being held in a second bag holder (58) having a bottom surface (22) and a draining opening (24) formed at a lowest point of the bottom surface (22), the bottom portion of the recirculation bag (52) resting on the bottom surface (22) of the second bag holder (58) such that the outlet of the recirculation bag (52) is located at the draining opening (24) of the second bag holder (58).

12. The device assembly (10) according to claim 11, **characterized in that** the second bag holder (58) includes a double-walled containment (18), the second filtration unit further comprising a temperature control system for controlling the temperature of the medium in the recirculation bag (52), the temperature control system including a heat transfer fluid flowing through the double-walled containment (18) of the second bag holder (58).

13. The device assembly (10) according to any of claims 10 to 12, **characterized in that** a conductivity sensor (68) is arranged in the recirculation bag (52).

14. The device assembly (10) according to any of claims 10 to 13, **characterized in that** the single-use loop-assembly includes controllable valves (80), preferably pinch valves.

15. The device assembly (10) according to any of the preceding claims, **characterized in that** a dip tray (82) is arranged below the single-use loop-assembly, the dip tray (82) having a bottom surface (22) and a draining means formed at a lowest point of the bottom surface (22).

16. The device assembly (10) according to any of the preceding claims, **characterized in that** the second filtration unit comprises at least one cross-flow filter cassette (72) held in a filter press (74).

17. The device assembly (10) according to any of the preceding claims, **characterized in that** the control unit (28) controls at least one of the following:
- temperature of a heat transfer fluid flowing through a double-walled containment (18) of the first bag holder (14);
- temperature of a heat transfer fluid flowing through a double-walled containment (18) of the second bag holder (58);
- controllable valves (80) of the single-use loop-assembly;
- a transfer pump (54) of the conjugation unit for transferring medium from the conjugation unit through the first filtration unit to the second filtration unit;
- a recirculation pump (76) of the single-use loop-assembly.

18. The device assembly (10) according to any of the preceding claims, **characterized in that** all components of the device assembly (10), except for an operating panel (84) coupled to the control unit (28), are mounted on a trolley (12).

## Patentansprüche

1. Vorrichtungsanordnung (10) zum Produzieren von Biokonjugaten, insbesondere Antikörpermedikamentenkonjugaten, wobei die Vorrichtungsanordnung (10) Folgendes umfasst
eine Konjugationseinheit zum Durchführen einer Biokonjugationsreaktion in einem Medium,
eine erste Filtrationseinheit zum Trennen von Präzipitaten und/oder Agglomeraten,
eine zweite Filtrationseinheit zum Durchführen eines Ultrafiltrations- und/oder eines Diafiltrationsprozesses,
wobei die erste Filtrationseinheit in einem Strömungspfad zwischen der Konjugationseinheit und der zweiten Filtrationseinheit angeordnet ist;
wobei die Vorrichtungsanordnung (10) ferner eine einzelne Steuereinheit (28) zum Steuern der Übertragung eines Mediums von der Konjugationseinheit durch die erste Filtrationseinheit zur zweiten Filtrationseinheit und zum Steuern des Ultrafiltrations- und/oder des Diafiltrationsprozesses umfasst,
**dadurch gekennzeichnet, dass** die zweite Filtrationseinheit eine vorausgelegte Einwegschleifenanordnung zum Rezirkulieren des Mediums im Ultrafiltrations- und/oder im Diafiltrationsprozess umfasst, die an einer Basisstruktur der Vorrichtungsanordnung (10) als Ganzes montiert und/oder von derselben demontiert werden kann.

2. Vorrichtungsanordnung (10) nach Anspruch 1, **dadurch gekennzeichnet, dass** eine erste Gruppe von Komponenten der Vorrichtungsanordnung (10), die mit dem Medium in Kontakt kommen, als Einwegkomponenten ausgelegt sind, die aus einem oder mehreren Materialien einer ersten Gruppe von Materialien bestehen, die aus Folgendem besteht: hochdichtes Polyethylen (HDPE); Polyamid (PA); Polybutylenterephthalat (PBT); Polytetrafluorethylen (PTFE); Polypropylen (PP); Polyethylenterephthalat (PET); Keramik; Glas; und dadurch, dass eine zweite Gruppe von Komponenten der Vorrichtungsanordnung (10), die mit dem Medium in Kontakt kommen, als Einwegkomponenten ausgelegt sind, die aus einem oder mehreren der folgenden Materialien bestehen: niedrigdichtes Polyethylen (LDPE); (verstärktes) platinvernetztes Silikon (Si(Pt)); thermoplastisches Elastomer (TPE); thermoplastisches Polyolefin (TPO); thermoplastisches Vulcanisat (TPV); Ethylenpropylendienmonomerkautschuk (EPDM); Silikon; einem Material aus der ersten Gruppe von Materialien.

3. Vorrichtungsanordnung (10) nach Anspruch 1 oder 2, **dadurch gekennzeichnet, dass** die Konjugationseinheit einen flexiblen Konjugationsbeutel (16) und einen ersten Beutelhalter (14), der den Konjugationsbeutel (16) hält, umfasst.

4. Vorrichtungsanordnung (10) nach Anspruch 3, **dadurch gekennzeichnet, dass** der Konjugationsbeutel (16) einen Bodenabschnitt und einen darin gebildeten Auslass aufweist, wobei der erste Beutelhalter (14) eine Bodenfläche (22) und eine Abflussöffnung (24) aufweist, die an einer niedrigsten Stelle der Bodenfläche (22) gebildet ist, wobei der Bodenabschnitt des Konjugationsbeutels (16) auf der Bodenfläche (22) des ersten Beutelhalters (14) ruht, derart, dass sich der Auslass des Konjugationsbeutels (16) an der Abflussöffnung (24) des ersten Beutelalters (14) befindet.

5. Vorrichtungsanordnung (10) nach Anspruch 3 oder 4, **dadurch gekennzeichnet, dass** der erste Beutelhalter (14) ein doppelwandiges Behältnis (18) beinhaltet, wobei die Konjugationseinheit ferner ein Temperatursteuersystem zum Steuern der Temperatur des Mediums im Konjugationsbeutel (16) umfasst, wobei das Temperatursteuersystem ein Wärmeübertragungsfluid beinhaltet, das durch das doppelwandige Behältnis (18) des ersten Beutelhalters (14) strömt.

6. Vorrichtungsanordnung (10) nach einem der Ansprüche 4 bis 5, **dadurch gekennzeichnet, dass** im Konjugationsbeutel (16) ein pH-Sensor (48) angeordnet ist.

7. Vorrichtungsanordnung (10) nach einem der vorhergehenden Ansprüche, **dadurch gekennzeichnet, dass** die Konjugationseinheit ein steuerbares Abflussventil (32) umfasst und die erste Filtrationseinheit eine Förderpumpe (54) zum Fördern des Mediums von der Konjugationseinheit durch die erste Filtrationseinheit zur zweiten Filtrationseinheit umfasst.

8. Vorrichtungsanordnung (10) nach einem der vorhergehenden Ansprüche, **dadurch gekennzeichnet, dass** die erste Filtrationseinheit eine Filtervorrichtung (56) mit einer Membran umfasst, die eine Porengröße von weniger als 0,8 µm, vorzugsweise ungefähr 0,2 µm oder weniger aufweist.

9. Vorrichtungsanordnung (10) nach einem der vorhergehenden Ansprüche, **dadurch gekennzeichnet, dass** die Einwegschleifenanordnung als Ganzes zusammen mit einem Einwegkonjugationsbeutel (16) und einem Einwegrezirkulationsbeutel (52) und vorzugsweise anderen Einwegkomponenten der Vorrichtungsanordnung (10) an eine Basisstruktur der Vorrichtungsanordnung (10) montiert und/oder von derselben demontiert werden kann.

10. Vorrichtungsanordnung (10) nach einem der vorhergehenden Ansprüche, **dadurch gekennzeichnet, dass** die Einwegschleifenanordnung eine Rezirkulationspumpe (76), mindestens einen Sensor (78), Leitungen und Verbinder (46) beinhaltet.

11. Vorrichtungsanordnung (10) nach Anspruch 10, **dadurch gekennzeichnet, dass** der Rezirkulationsbeutel (52) einen Bodenabschnitt und einen darin gebildeten Auslass aufweist, wobei der Rezirkulationsbeutel (52) in einem zweiten Beutelhalter (58) gehalten wird, der eine Bodenfläche (22) und eine Abflussöffnung (24) aufweist, die an einer niedrigsten Stelle der Bodenfläche (22) gebildet ist, wobei der Bodenabschnitt des Rezirkulationsbeutels (52) auf der Bodenfläche (22) des zweiten Beutelhalters (58) ruht, derart, dass sich der Auslass des Rezirkulationsbeutels (52) an der Abflussöffnung (24) des zweiten Beutelalters (58) befindet.

12. Vorrichtungsanordnung (10) nach Anspruch 11, **dadurch gekennzeichnet, dass** der zweite Beutelhalter (58) ein doppelwandiges Behältnis (18) beinhaltet, wobei die zweite Filtrationseinheit ferner ein Temperatursteuersystem zum Steuern der Temperatur des Mediums im Rezirkulationsbeutel (52) umfasst, wobei das Temperatursteuersystem ein Wärmeübertragungsfluid beinhaltet, das durch das doppelwandige Behältnis (18) des zweiten Beutelhalters (58) strömt.

13. Vorrichtungsanordnung (10) nach einem der Ansprüche 10 bis 12, **dadurch gekennzeichnet, dass** im Rezirkulationsbeutel (52) ein Leitfähigkeitssensor (68) angeordnet ist.

14. Vorrichtungsanordnung (10) nach einem der Ansprüche 10 bis 13, **dadurch gekennzeichnet, dass** die Einwegschleifenanordnung steuerbare Ventile (80), vorzugsweise Quetschventile beinhaltet.

15. Vorrichtungsanordnung (10) nach einem der vorhergehenden Ansprüche, unter der Einwegschleifenanordnung eine Tropfschale (82) angeordnet ist, wobei die Tropfschale (82) eine Bodenfläche (22) und ein Abflussmittel aufweist, das an der niedrigsten Stelle der Bodenfläche (22) gebildet ist.

16. Vorrichtungsanordnung (10) nach einem der vorhergehenden Ansprüche, **dadurch gekennzeichnet, dass** die zweite Filtrationseinheit mindestens eine Querstromfilterkassette (72) umfasst die in einer Filterpresse (74) gehalten wird.

17. Vorrichtungsanordnung (10) nach einem der vorhergehenden Ansprüche, **dadurch gekennzeichnet, dass** die Steuereinheit (28) mindestens eines von Folgendem steuert:
- Temperatur eines Wärmeübertragungsfluids, das durch ein doppelwandiges Behältnis (18) des ersten Beutelhalters (14) strömt;
- Temperatur eines Wärmeübertragungsfluids, das durch ein doppelwandiges Behältnis (18) des zweiten Beutelhalters (58) strömt;
- steuerbare Ventile (80) der Einwegschleifenanordnung;
- eine Förderpumpe (54) der Konjugationseinheit zum Übertragen eines Mediums von der Konjugationseinheit durch die erste Filtrationseinheit zur zweiten Filtrationseinheit;
- eine Rezirkulationspumpe (76) der Einwegschleifenanordnung.

18. Vorrichtungsanordnung (10) nach einem der vorhergehenden Ansprüche, **dadurch gekennzeichnet, dass** alle Komponenten der Vorrichtungsanordnung (10) abgesehen von einem Bedienpaneel (84), das an die Steuereinheit (28) gekoppelt ist, auf einem Wagen (12) montiert sind.

## Revendications

1. Ensemble de dispositif (10) pour la production de bio-conjugués, en particulier des conjugués anticorps-médicament, l'ensemble de dispositif (10) comprenant :
une unité de conjugaison pour réaliser une réaction de bio-conjugaison dans un milieu,
une première unité de filtration pour séparer des précipités et/ou des agglomérats,
une seconde unité de filtration pour réaliser un processus d'ultrafiltration et/ou de diafiltration,
la première unité de filtration étant agencée dans une trajectoire d'écoulement entre l'unité de conjugaison et la seconde unité de filtration ;
l'ensemble de dispositif (10) comprenant en outre une seule unité de commande (28) pour commander le transfert de milieu de l'unité de conjugaison, en passant par la première unité de filtration jusqu'à la seconde unité de filtration et pour commander le processus d'ultrafiltration et/ou de diafiltration,
**caractérisé en ce que** la seconde unité de filtration comprend un ensemble boucle à usage unique préconfiguré pour faire recirculer le milieu dans le processus d'ultrafiltration et/ou de diafiltration qui peut être monté sur et/ou démonté d'une structure de base de l'ensemble de dispositif (10) dans son ensemble.

2. Ensemble de dispositif (10) selon la revendication 1, **caractérisé en ce qu'**un premier groupe de composants de l'ensemble de dispositif (10) venant en contact avec le milieu est configuré comme étant des composants à usage unique réalisés à partir d'un ou de plusieurs matériaux d'un premier groupe de matériaux comprenant : le polyéthylène haute densité (HDPE) ; le polyamide (PA) ; le polybutylène téréphtalate (PBT) ; le polytétrafluoroéthylène (PTFE) ; le polypropylène (PP) ; le polyéthylène téréphtalate (PET) ; la céramique ; le verre ; et **en ce qu'**un second groupe de composants de l'ensemble de dispositif (10) venant en contact avec le milieu est configuré comme étant des composants à usage unique réalisés à partir d'un ou des plusieurs matériaux suivants : le polyéthylène faible densité (LDPE) ; la silicone vulcanisé au platine (renforcé) (Si(Pt)) ; un élastomère thermoplastique (TPE) ; la polyoléfine thermoplastique (TPO) ; un vulcanisat thermoplastique (TPV) ; un caoutchouc de monomère de type éthylène, propylène, diène (EPDM) ; la silicone ; un matériau du premier groupe de matériaux.

3. Ensemble de dispositif (10) selon la revendication 1 ou 2, **caractérisé en ce que** l'unité de conjugaison comprend un sac de conjugaison souple (16) et un premier support de sac (14) maintenant le sac de conjugaison (16).

4. Ensemble de dispositif (10) selon la revendication 3, **caractérisé en ce que** le sac de conjugaison (16) a une partie inférieure et une sortie formée dans cette dernière, le premier support de sac (14) ayant une surface inférieure (22) et une ouverture d'évacuation (24) formée au point le plus bas de la surface inférieure (22), la partie inférieure du sac de conjugaison (16) s'appuyant sur la surface inférieure (22) du premier support de sac (14) de sorte que la sortie du sac de conjugaison (16) est positionnée au niveau de l'ouverture d'évacuation (24) du premier support de sac (14).

5. Ensemble de dispositif (10) selon la revendication 3 ou 4, **caractérisé en ce que** le premier support de sac (14) comprend un confinement à double paroi (18), l'unité de conjugaison comprenant en outre un système de contrôle de température pour contrôler la température du milieu dans le sac de conjugaison (16), le système de contrôle de température comprenant un fluide de transfert de chaleur s'écoulant à travers le confinement à double paroi (18) du premier support de sac (14).

6. Ensemble de dispositif (10) selon l'une quelconque des revendications 4 à 5, **caractérisé en ce qu'**un capteur de pH (48) est agencé dans le sac de conjugaison (16).

7. Ensemble de dispositif (10) selon l'une quelconque des revendications précédentes, **caractérisé en ce que** l'unité de conjugaison comprend une valve d'évacuation (32) contrôlable et la première unité de filtration comprend une pompe de transfert (54) pour transférer le milieu de l'unité de conjugaison, en passant par la première unité de filtration, jusqu'à la seconde unité de filtration.

8. Ensemble de dispositif (10) selon l'une quelconque des revendications précédentes, **caractérisé en ce que** la première unité de filtration comprend un dispositif de filtre (56) avec une membrane ayant une taille de pore inférieure à 0,8 µm, de préférence d'environ 0,2 µm ou moins.

9. Ensemble de dispositif (10) selon l'une quelconque des revendications précédentes, **caractérisé en ce que** l'ensemble boucle à usage unique peut être monté sur et/ou démonté d'une structure basique de l'ensemble de dispositif (10) dans son ensemble, conjointement avec un sac de conjugaison (16) à usage unique et un sac de recirculation (52) à usage unique et de préférence d'autres composants à usage unique de l'ensemble de dispositif (10).

10. Ensemble de dispositif (10) selon l'une quelconque des revendications précédentes, **caractérisé en ce que** l'ensemble boucle à usage unique comprend une pompe de recirculation (76), au moins un capteur (78), des tuyaux et des connecteurs (46).

11. Ensemble de dispositif (10) selon la revendication 10, **caractérisé en ce que** le sac de recirculation (52) a une partie inférieure et une sortie formée dans cette dernière, le sac de recirculation (52) étant maintenu dans un second support de sac (58) ayant une surface inférieure (22) et une ouverture d'évacuation (24) formée au niveau du point le plus bas de la surface inférieure (22), la partie inférieure du sac de recirculation (52) s'appuyant sur la surface inférieure (22) du second support de sac (58) de sorte que la sortie du sac de recirculation (52) est positionnée au niveau de l'ouverture d'évacuation (24) du second support de sac (58).

12. Ensemble de dispositif (10) selon la revendication 11, **caractérisé en ce que** le second support de sac (58) comprend un confinement à double paroi (18), la seconde unité de filtration comprenant en outre un système de contrôle de température pour contrôler la température du milieu dans le sac de recirculation (52), le système de contrôle de température comprenant un fluide de transfert de chaleur s'écoulant à travers le confinement à double paroi (18) du second support de sac (58).

13. Ensemble de dispositif (10) selon l'une quelconque des revendications 10 à 12, **caractérisé en ce qu'**un capteur de conductivité (68) est agencé dans le sac de recirculation (52).

14. Ensemble de dispositif (10) selon l'une quelconque des revendications 10 à 13, **caractérisé en ce que** l'ensemble boucle à usage unique comprend des valves contrôlables (80), de préférence des robinets à manchon déformable.

15. Ensemble de dispositif (10) selon l'une quelconque des revendications précédentes, **caractérisé en ce qu'**un châssis de trempage (82) est agencé au-dessous de l'ensemble boucle à usage unique, le châssis de trempage (82) ayant une surface inférieure (22) et un moyen d'évacuation formé au niveau du point le plus bas de la surface inférieure (22).

16. Ensemble de dispositif (10) selon l'une quelconque des revendications précédentes, **caractérisé en ce que** la seconde unité de filtration comprend au moins une cassette de filtre à flux croisés (72) maintenue dans un filtre-presse (74).

17. Ensemble de dispositif (10) selon l'une quelconque des revendications précédentes, **caractérisé en ce que** l'unité de commande (28) commande au moins l'un des éléments suivants :
la température d'un fluide de transfert de chaleur s'écoulant par un confinement à double paroi (18) du premier support de sac (14) ;
la température d'un fluide de transfert de chaleur s'écoulant à travers un confinement à double paroi (18) du second support de sac (58) ;
des valves contrôlables (80) de l'ensemble boucle à usage unique ;
une pompe de transfert (54) de l'unité de conjugaison pour transférer le milieu de l'unité de conjugaison, en passant par la première unité de filtration, jusqu'à la seconde unité de filtration ;
une pompe de recirculation (76) de l'ensemble boucle à usage unique.

18. Ensemble de dispositif (10) selon l'une quelconque des revendications précédentes, **caractérisé en ce que** tous les composants de l'ensemble de dispositif (10), excepté pour un panneau de commande (84) couplé à l'unité de commande (28), sont montés sur un chariot (12).
